# EUROPEAN PATENT APPLICATION

(11) **EP 4 786 977 A1**
(43) Date of publication of application: **05.08.2026**
(21) Application number: 25155815.1
(22) Date of filing: 04.02.2025
(51) Int. Cl.: G01N 33/483

(54) **METHOD AND SYSTEM FOR IDENTIFYING BIOMARKERS OF A HEALTH CONDITION**

(71) Applicant: Imec VZW, 3001 Leuven (BE); Katholieke Universiteit Leuven, 3000 Leuven (BE); VIB VZW, 9052 Zwijnaarde (BE)
(72) Inventor: LAMBRECHTS, Dennis, 3018 Wijgmaal (BE); BRAEKEN, Dries, 3401 Waasmont (BE); CALATAYUD ARISTOY, Carles, 3000 Leuven (BE); VERSTREKEN, Patrik, 3052 Blanden (BE)
(74) Representative: Winger

(57) **Abstract**

A method for identifying biomarkers of a health condition, comprising: providing a first neuronal circuit with neuronal cells from a first individual organism on a multi-electrode array, applying stimuli to the circuit, obtaining electrophysiological recordings, deriving cell-level and/or circuit-level features from the recordings, associating derived features with presence or absence of the health condition, comparing associated data with reference data, and determining if reference features or derived features are biomarkers for the condition.

## Description

### Field of the Invention

The present invention relates to the field of neurobiological studies, and more specifically to electrophysiological analysis of neuronal circuits.

### Background of the Invention

Neurodegenerative diseases, such as Parkinson's disease, Alzheimer's disease, and others, pose significant challenges to global health due to their progressive nature and the lack of effective treatments. Early identification of individuals at risk of developing these disorders is advantageous for improving patient outcomes and facilitating research into disease-modifying interventions. Detecting these conditions at preclinical or prodromal stages could enable timely therapeutic strategies when interventions may have the greatest potential for success.

Despite the importance of early detection, there remains a substantial lack of specific biomarkers of the initial phases of neurodegenerative diseases. Current methods often rely on clinical assessments and imaging techniques that may not capture subtle, early changes associated with disease onset. This limitation hinders the ability to implement personalized treatment approaches and to recruit appropriate participants for clinical trials aimed at testing new therapeutic agents.

In the quest to better understand neurodegenerative conditions, in vitro disease models have gained attention. These models utilize patient-specific induced neurons and organoids derived from cellular reprogramming technologies. They offer a controlled environment to study cellular mechanisms underlying disease processes. However, a significant challenge with these models is their difficulty in replicating clinically relevant hallmarks of neurodegenerative diseases. This gap limits the translational potential of findings obtained from such systems.

Electrophysiological characterization techniques provide a window into the functional properties of neuronal networks. Advanced tools, such as complementary-metal-oxide-semiconductor multi-electrode array (CMOS-MEA) chips, have enabled high-resolution recording of neuronal activity. These chips possess a high electrode density, allowing for detailed analysis from single-cell level dynamics to population-level behaviors. Nevertheless, existing data analysis strategies do not fully capture the complexities and nuances associated with disease-related neuronal dysfunctions.

Given these challenges, there is a clear need for further advancements in the field.

### Summary of the Invention

It is an object of embodiments of the present invention to provide methods and systems for identifying biomarkers of a health condition using organism-specific neuronal circuits. This objective is accomplished by the aspects of the present invention.

In a first aspect, the present invention relates to a method for identifying (potential) biomarkers of a health condition. The method comprises (a) providing a first neuronal circuit for establishing neuronal activity therein, wherein all cells within the first neuronal circuit stem from a same first individual organism. The first neuronal circuit is provided on a multi-electrode array for recording neuronal activity, has a composition that comprises at least two different types of cells communicating with each other, the composition comprising at least one type of neuronal cell, and has a structure formed by a specific spatial arrangement of its cells on the multi-electrode array. The method further comprises (b) applying one or more stimuli to the first neuronal circuit, (c) obtaining one or more electrophysiological recordings from the first neuronal circuit in response to the stimuli, (d) deriving one or more cell-level electrophysiological features and/or one or more circuit-level electrophysiological features from at least one of the electrophysiological recordings, (e) associating at least one derived electrophysiological feature with the presence or absence of said health condition thereby forming associated data, (f) comparing the associated data with at least one reference associated data formed of the association of a reference electrophysiological feature with the presence or absence of said health condition, wherein at least one of the associated data and the reference associated data is formed of an association of at least one electrophysiological feature with the presence of said health condition, and (g) determining from that comparison whether at least one of the reference electrophysiological features or at least one of the derived one or more cell-level electrophysiological features and/or one or more circuit-level electrophysiological features is a (potential) biomarker specific for the health condition.

In embodiments, the at least two different types of cells may comprise two different types of electrogenic cells.

In embodiments, the at least two different types of cells may comprise two different types of neuronal cells.

In embodiments, the at least two different types of cells may consist of at least two different types of neuronal cells.

In embodiments of the first aspect, the present invention relates to a method for identifying (potential) biomarkers of a health condition. The method comprises (a) providing a first neuronal circuit for establishing neuronal activity therein, wherein all neuronal cells within the first neuronal circuit stem from a same first individual organism. The first neuronal circuit is provided on a multi-electrode array for recording neuronal activity, has a composition that comprises at least two different types of communicating neuronal cells, and has a structure formed by a specific spatial arrangement of its neuronal cells on the multi-electrode array. The method further comprises (b) applying one or more stimuli to the first neuronal circuit, (c) obtaining one or more electrophysiological recordings from the first neuronal circuit in response to the stimuli, (d) deriving one or more cell-level electrophysiological features and/or one or more circuit-level electrophysiological features from at least one of the electrophysiological recordings, (e) associating at least one derived electrophysiological feature with the presence or absence of said health condition thereby forming associated data, (f) comparing the associated data with at least one reference associated data formed of the association of a reference electrophysiological feature with the presence or absence of said health condition, wherein at least one of the associated data and the reference associated data is formed of an association of at least one electrophysiological feature with the presence of said health condition, and (g) determining from that comparison whether at least one of the reference electrophysiological features or at least one of the derived one or more cell-level electrophysiological features and/or one or more circuit-level electrophysiological features is a (potential) biomarker specific for the health condition.

In embodiments, deriving one or more cell-level electrophysiological features and/or one or more circuit-level electrophysiological features may comprise at least one of deriving one or more circuit-level electrophysiological features by analyzing spatial variations in electrophysiological activity across the neuronal circuit as recorded from different electrodes of the multi-electrode array, deriving one or more cell-level electrophysiological features and/or one or more circuit-level electrophysiological features by analyzing temporal variations in electrophysiological activity over a plurality of time points, and deriving one or more circuit-level electrophysiological features by analyzing spatiotemporal patterns of electrophysiological activity resulting from the combination of spatial and temporal variations. This allows for comprehensive characterization of the neuronal circuit activity at both the single-cell and network levels.

In embodiments, associating the at least one derived electrophysiological feature with the presence or absence of said health condition may further comprise associating the at least one derived electrophysiological feature with one or more confounding factors present in the individual organism from whom the neuronal circuit stems from, thereby forming the associated data, and wherein the reference associated data is further formed from the association of the reference electrophysiological feature with said one or more confounding factors. This enables accounting for potential confounding variables that may influence the electrophysiological features.

In embodiments, the performance of comparing the associated data with at least one reference associated data and determining whether the reference electrophysiological feature, said at least one of the derived one or more cell-level electrophysiological features and/or one or more circuit-level electrophysiological features is a biomarker specific for the health condition may comprise using generative models and/or latent space models for accounting for the confounding factors. This provides a robust approach for handling confounding variables in the biomarker identification process.

In embodiments, the generative models may include variational autoencoder schemes. This offers a powerful framework for modeling complex data distributions and accounting for confounding factors.

In embodiments, the confounding factors may include organism-centered data selected from the group consisting of genetic information, medical records, concurrent diseases, use of pharmaceuticals, and diet. This allows for a comprehensive consideration of various factors that may influence the electrophysiological features and biomarker identification.

In embodiments, one or more of the at least one reference associated data may be obtained in the same way as the associated data but by providing a neuronal circuit stemming from an individual different from the first individual. This allows for a controlled comparison between neuronal circuits from different individuals.

In embodiments, one or more of the at least one reference associated data may be obtained by the performance of providing a first neuronal circuit for establishing neuronal activity therein, applying one or more stimuli to the first neuronal circuit, obtaining one or more electrophysiological recordings from the first neuronal circuit in response to the stimuli, deriving one or more cell-level electrophysiological features and/or one or more circuit-level electrophysiological features from at least one of the electrophysiological recordings, and associating at least one derived electrophysiological feature with the presence or absence of said health condition thereby forming associated data on one or more further neuronal circuits. Each of the further neuronal circuits may have all its cells stemming from a same further individual organism different from the first organism, be provided on a multi-electrode array for recording neuronal activity, have the same composition as the first neuronal circuit in terms of the number of different types of cells communicating with each other, and have the same structure as the first neuronal circuit formed by the same specific spatial arrangement of its cells on the multi-electrode array. For said one or more of the at least one further neuronal circuit, applying one or more stimuli to the first neuronal circuit may use the same one or more stimuli. This enables a controlled comparison between neuronal circuits from different individuals while maintaining consistency in the experimental conditions.

In embodiments, deriving one or more cell-level electrophysiological features and/or one or more circuit-level electrophysiological features from at least one of the electrophysiological recordings may comprise performing a preliminary electrophysiological recording of the first neuronal circuit before applying the one or more stimulus, and deriving the features from said at least one of the electrophysiological recordings and from said preliminary electrophysiological recordings. This enables capturing both the baseline activity and the evoked response of the neuronal circuit.

In embodiments, the one or more preliminary electrophysiological recordings of the first neuronal circuit may be performed before applying the one or more stimulus, wherein said preliminary electrophysiological recordings are used to detect areas on the multi-electrode array where electrogenic cells (e.g., neurons) exhibit correlated firing patterns indicative of functional connectivity, and wherein step (c) comprises obtaining one or more electrophysiological recordings from the first neuronal circuit at these areas.

In embodiments, the structure of the neuronal circuit may mimic a neural circuit associated with the health condition. This provides a biologically relevant model for studying the specific health condition.

In embodiments, each of the one or more stimuli may be selected from the group consisting of an acoustic stimulus, an optical stimulus such as an optogenetic stimulus, an electrical stimulus, a chemical stimulus such as a biochemical stimulus, an electroporation of a genetic factor into a cell (e.g., into a neuronal cell), or a combination thereof. This allows for diverse stimulation modalities to probe the neuronal circuit's response.

In embodiments, the chemical stimulus may be an agonist, an antagonist, or a modulator. This enables targeted manipulation of specific neuronal receptors or signaling pathways.

In embodiments, the cells may be human or mammalian cells. This ensures the relevance of the model to human health conditions.

In embodiments, the cells may be electrogenic (i.e., generating action potentials) or non-electrogenic (i.e., that do not generate action potentials). Non-electrogenic cells can communicate with each other and/or with electrogenic cells via chemical, electrical, or mechanical signaling. Examples of electrogenic cells are neurons, cardiac myocytes, muscle cells, sensory receptor cells, and endocrine cells.

In embodiments, the cells may be derived from induced pluripotent stem cells (iPSCs). This allows for the generation of patient-specific neuronal circuits.

In embodiments, the cells may be reprogrammed from fibroblasts or blood cells derived from a single individual organism. This enables the creation of personalized neuronal circuits from easily accessible patient samples.

In embodiments, the at least two different types of cells comprise at least two different types of neuronal cells selected from excitatory neurons, inhibitory neurons, and modulatory neurons, and preferably comprise excitatory neurons and modulatory neurons. This captures the key neuronal subtypes involved in neural circuit function.

In embodiments, the first neuronal circuit may be supported by glial cells. This provides a more physiologically relevant environment for the neuronal cells.

In embodiments, the composition of the circuit may comprise at least three different types of cells. This allows for more complex and realistic neuronal circuit architectures.

In embodiments, the composition of the circuit may comprise or consist of at least three different types of neuronal cells.

In embodiments, the at least three different types of neuronal cells may comprise excitatory neurons, inhibitory neurons, and modulatory neurons. This captures the main neuronal subtypes involved in neural circuit function.

In embodiments, the excitatory neurons may comprise cortical neurons. This reflects the predominant excitatory neuron type in the cerebral cortex.

In embodiments, the inhibitory neurons may comprise striatal neurons. This reflects the predominant inhibitory neuron type in the striatum.

In embodiments, the modulatory neurons may comprise dopaminergic neurons. This captures a key neuromodulatory neuron type involved in various brain functions and disorders.

In embodiments, said first individual organism may carry a genetic mutation associated with the health condition. This allows for the study of the impact of specific genetic risk factors on neuronal circuit function.

In embodiments, the method may further comprise a step, before providing a first neuronal circuit for establishing neuronal activity therein, of forming the specific spatial arrangement of the cells by forming a physical barrier to create different compartments on the multi-electrode array, introducing the different cell types in the different compartments, and optionally removing said physical barrier. This enables precise control over the spatial organization of the neuronal circuit.

In embodiments, the method may further comprise a step, before providing a first neuronal circuit for establishing neuronal activity therein, of forming the specific spatial arrangement of the cells by sequentially introducing different types of cells onto the multi-electrode array. This allows for the creation of defined neuronal circuit architectures.

In embodiments, the method may further comprise a step, before providing a first neuronal circuit for establishing neuronal activity therein, of forming the specific spatial arrangement of the cells by introducing a first cell type onto the multi-electrode array, electroporating some of the introduced cells with genetic factors that confer antibiotic resistance, exposing the introduced cells to said antibiotic thereby eliminating the non-electroporated cells, introducing one or more additional cell types onto the multi-electrode array, and optionally repeating the electroporation and antibiotic exposure steps for each additional cell type, thereby selectively eliminating undesired cells and forming a specific spatial arrangement of the cells. This enables the selective removal of unwanted cell types and the creation of defined neuronal circuit architectures.

In embodiments, the method may further comprise a step, before providing a first neuronal circuit for establishing neuronal activity therein, of forming the specific spatial arrangement of the cells by directing different cell types to different locations on the multielectrode array by using fluidic, acoustic, and/or chemical isolation techniques. This allows for precise spatial patterning of the neuronal circuit.

In embodiments, the method may further comprise a step, before providing a first neuronal circuit for establishing neuronal activity therein, of forming the specific spatial arrangement of the cells by introducing undifferentiated cells on the multielectrode array, and electroporating the introduced undifferentiated cells with genetic factors that promote trans-differentiation of the undifferentiated cells into the desired neuronal types. This enables the in situ generation of specific neuronal subtypes within the circuit.

In embodiments, the multi-electrode array may be a complementary-metal-oxide-semiconductor multi-electrode array chip. This provides a high-density, high-resolution platform for recording neuronal activity.

In embodiments, the multi-electrode array may be adapted for single-cell level recordings of neuronal activity. This allows for the precise characterization of individual neuronal responses.

In embodiments, the multi-electrode array may have an electrode density exceeding 1000 electrodes per mm². This enables high-resolution mapping of neuronal activity across the circuit.

In embodiments, the multi-electrode array electrodes may be individually addressable. This allows for targeted stimulation and recording from specific regions of the neuronal circuit.

In embodiments, the multi-electrode array electrodes may have their largest lateral dimension measuring from 2 to 500 µm, preferably from 2 to 50 µm, more preferably from 2 to 25 µm. This enables the recording of neuronal activity at different spatial scales.

In embodiments, the electrophysiological features may comprise cell-level features derived from action potential waveform characteristics. This allows for the characterization of individual neuronal firing properties.

In embodiments, the electrophysiological features may include circuit-level features derived from spike train data. This enables the analysis of network-level activity patterns.

In embodiments, the electrophysiological features may be selected from network synchrony, rhythmicity, connectivity, and firing patterns. This captures key aspects of neuronal circuit dynamics.

In embodiments, the electrophysiological features may be derived using a spike sorting algorithm to decompose the electrophysiological recording data into contributions from individual electrogenic cells (e.g., neurons). This enables the isolation of single-unit activity from the multi-electrode array recordings.

In embodiments, the electrophysiological features may be derived using a biophysical model-based fitting process to determine activation time points. This allows for the precise estimation of neuronal firing times.

In embodiments, the confounding factors may comprise batch-specific variability and/or experimental covariates. This enables accounting for potential sources of experimental noise or bias.

In embodiments, the performance of comparing the associated data with at least one reference associated data and determining whether the reference electrophysiological feature, said at least one of the derived one or more cell-level electrophysiological features and/or one or more circuit-level electrophysiological features is a biomarker specific for the health condition may comprise using mutual nearest neighbor models. This provides a robust approach for identifying biomarkers from high-dimensional data.

In embodiments, the performance of comparing the associated data with at least one reference associated data and determining whether the reference electrophysiological feature, said at least one of the derived one or more cell-level electrophysiological features and/or one or more circuit-level electrophysiological features is a biomarker specific for the health condition may comprise applying machine learning classification algorithms selected from the group consisting of decision trees, logistic regression, principal component analysis, naive Bayes models, support vector machines, random forests, and nearest neighbor models. This allows for the use of powerful computational tools for biomarker identification.

In embodiments, providing a first neuronal circuit for establishing neuronal activity therein, applying one or more stimuli to the first neuronal circuit, obtaining one or more electrophysiological recordings from the first neuronal circuit in response to the stimuli, deriving one or more cell-level electrophysiological features and/or one or more circuit-level electrophysiological features from at least one of the electrophysiological recordings, associating at least one derived electrophysiological feature with the presence or absence of said health condition thereby forming associated data, comparing the associated data with at least one reference associated data formed of the association of a reference electrophysiological feature with the presence or absence of said health condition, and determining from that comparison whether the reference electrophysiological feature, said at least one of the derived one or more cell-level electrophysiological features and/or one or more circuit-level electrophysiological features is a biomarker specific for the health condition may be repeated sequentially in an iterative manner for different neuronal circuits. The method may further comprise adjusting one or more steps of the method in a currently analyzed neuronal circuit, based on the output of one or more steps of the method in one or more previously analyzed neuronal circuits. This enables an adaptive and iterative approach to biomarker identification, where insights from previous analyses inform subsequent experiments.

In embodiments, the method may further comprise a step, between deriving one or more cell-level electrophysiological features and/or one or more circuit-level electrophysiological features from at least one of the electrophysiological recordings and associating at least one derived electrophysiological feature with the presence or absence of said health condition thereby forming associated data, of applying a feature selection step to reduce data dimensionality. This allows for the identification of the most informative features for biomarker discovery.

In embodiments, the method may be for identifying biomarkers of a health condition and classifying neuronal circuits. The method may further comprise obtaining electrophysiological recordings from new individual organisms neuronal circuits, extracting electrophysiological features from these recordings, comparing the extracted features to at least one biomarker identified by determining from the comparison of the associated data with at least one reference associated data whether the reference electrophysiological feature, said at least one of the derived one or more cell-level electrophysiological features and/or one or more circuit-level electrophysiological features is a biomarker specific for the health condition, and based on the comparison of the extracted features to at least one biomarker, classifying the new individual organism neuronal circuits into phenotypically different populations. This enables the application of the identified biomarkers for diagnostic or prognostic purposes.

In embodiments, the method may be for identifying biomarkers of a health condition and for phenotype atlas construction. The method may further comprise building an atlas of disease-associated electrophysiological phenotypes based on the identified biomarkers. This allows for the creation of a comprehensive resource for understanding the electrophysiological signatures of different health conditions.

In embodiments, the health condition may be a neurodegenerative disorder. This enables the identification of biomarkers.

In embodiments, the health condition may be a neurodegenerative disorder selected from Parkinson's disease, Alzheimer's disease, Huntington's disease, and amyotrophic lateral sclerosis. This allows identifying disease-specific biomarkers.

In a second aspect, the present invention relates to a system for identifying biomarkers of a health condition, the system comprising means adapted to execute the steps of the method of any embodiments of the first aspect.

In a third aspect, the present invention relates to a system for identifying biomarkers of a health condition, comprising:
(a) a multi-electrode array configured to support a neuronal circuit;
(b) a stimulation unit configured to apply one or more stimuli to the neuronal circuit to evoke neuronal activity therein;
(c) a recording unit configured to obtain electrophysiological recordings from the neuronal circuit in response to the stimuli;
(d) a computing unit configured to control the stimulation unit and the recording unit, derive one or more cell-level and/or circuit-level electrophysiological features from the electrophysiological recordings, associate each derived electrophysiological feature with the presence or absence of a health condition in the individual organism from which the neuronal circuit stem from and with one or more confounding factors, thereby forming associated data, and compare the associated data with at least one reference associated data formed of the association of a reference electrophysiological feature with the presence or absence of said health condition, wherein at least one of the associated data and the reference associated data is formed of an association of at least one electrophysiological feature with the presence of said health condition, determine from that comparison whether the reference electrophysiological feature, said at least one of the derived one or more cell-level electrophysiological features and/or one or more circuit-level electrophysiological features is a biomarker specific for the health condition.

In embodiments, the stimulation unit may be configured to apply one or more stimuli selected from the group consisting of an acoustic stimulus, an optical stimulus such as an optogenetic stimulus, an electrical stimulus, a chemical stimulus such as a biochemical stimulus, an electroporation of a genetic factor into a cell (e.g., into a neuronal cell), or a combination thereof. This enables evoking different types of neuronal activity.

In embodiments, the multi-electrode array may be a complementary-metal-oxide-semiconductor multi-electrode array chip. This provides high spatial resolution.

In embodiments, the multi-electrode array may be adapted for single-cell level recordings of neuronal activity. This allows deriving cell-level features.

In embodiments, the multi-electrode array may have an electrode density exceeding 1,000 electrodes per mm². This enables high-resolution mapping.

In embodiments, the multi-electrode array electrodes may be individually addressable. This allows targeted stimulation and recording.

In embodiments, the multi-electrode array electrodes may have their largest lateral dimension measuring from 2 to 500 µm, preferably from 2 to 50 µm, more preferably from 2 to 25 µm. This is optimal for interfacing with neurons.

In embodiments, the electrophysiological features may comprise cell-level features derived from action potential waveform characteristics. This provides information on cell firing properties.

In embodiments, the electrophysiological features may include circuit-level features derived from spike train data. This provides information on network dynamics.

In embodiments, the circuit-level features may be selected from network synchrony, rhythmicity, connectivity, and firing patterns. These are key aspects of circuit function.

In embodiments, the computing unit may be configured to use a spike sorting algorithm to decompose the electrophysiological recording data into contributions from individual electrogenic cells (e.g., neurons). This enables cell-level analysis.

In embodiments, the computing unit may be configured to use a biophysical model-based fitting process to determine activation timepoints. This is an alternative to spike sorting.

In embodiments, the computing unit may be configured to form and compare the associated data using generative models and/or latent space models for accounting for confounding factors. This enables robust comparisons.

In embodiments, the generative models may include variational autoencoder schemes. These are powerful unsupervised learning models.

In embodiments, the computing unit may be configured to form and compare the associated data using mutual nearest neighbor models. These can identify similar samples across modalities.

In embodiments, the computing unit may be configured to form and compare the associated data by applying machine learning classification algorithms selected from the group consisting of decision trees, logistic regression, principal component analysis, naive Bayes models, support vector machines, random forests, and nearest neighbor models. These are established classification techniques.

In embodiments, the computing unit may be configured to repeat the identification of biomarkers for different neuronal circuits, and may be further configured to adjust one or more steps of the method in a currently analyzed neuronal circuit, based on the output of one or more steps of the method in one or more previously analyzed neuronal circuits. This enables iterative optimization.

In embodiments, the computing unit may be configured to apply a feature selection step between the derivation of the features and the forming of the associated data to reduce data dimensionality during analysis. This facilitates classification.

In embodiments, the computing unit may be configured for, once biomarkers have been identified, comparing extracted features derived from an individual organism neuronal circuit to the identified biomarkers, and based on that comparison, classifying the individual organism neuronal circuit into a phenotypically specific population. This enables patient stratification.

In embodiments, the computing unit may be further configured for phenotype atlas construction by building an atlas of disease-associated electrophysiological phenotypes based on the identified biomarkers. This provides a reference for diagnosis.

In a fourth aspect, the present invention relates to a computer program comprising instructions to cause the system of any embodiments of the third aspect to execute the steps of the method of any embodiments of the first aspect.

In a fifth aspect, the present invention relates to a computer-readable medium having stored thereon the computer program of the fourth aspect.

It is an advantage of embodiments of the present invention that early detection of health conditions such as neurodegenerative disorders is facilitated through the identification of specific biomarkers in patient-specific neuronal circuits.

It is an advantage of embodiments of the present invention that the architecture of neuronal circuits on a chip can be built and controlled, allowing investigation of clinically relevant aspects of brain diseases.

It is an advantage of embodiments of the present invention that neuronal circuit dysfunctions can be studied, which is not possible using traditional single-phenotype cultures.

It is an advantage of embodiments of the present invention that high-resolution electrophysiological recordings can be obtained using high-density complementary-metal-oxide-semiconductor multi-electrode array (CMOS-MEA) chips.

It is an advantage of embodiments of the present invention that both single-cell and population-level characterizations can be conducted to compare healthy and diseased conditions.

It is an advantage of embodiments of the present invention that various techniques can be employed to control circuit architecture, increasing versatility in modeling different brain circuits.

It is an advantage of embodiments of the present invention that multimodal datasets, including patient-centered databases like genetics and medical records, can be integrated to account for confounding factors and enhance comparative analysis.

It is an advantage of embodiments of the present invention that an atlas of disease-associated electrophysiological phenotypes may be built, aiding in the classification and stratification of patients into different phenotypic populations.

It is an advantage of embodiments of the present invention that it could lead to personalized treatment approaches that can be developed and initiated at the earliest stages of disease when interventions are most effective.

It is an advantage of embodiments of the present invention that some limitations of conventional in vitro disease models may be overcome by providing clinically relevant data on neuronal circuit dysfunction.

It is an advantage of embodiments of the present invention that it helps discover biomarkers useful in the preclinical or prodromal phases of neurodegenerative diseases.

It is an advantage of embodiments of the present invention that quantitative comparative analysis between healthy and diseased conditions can be enabled by accounting for confounding factors and integrating multimodal datasets.

It is an advantage of embodiments of the present invention that the potential for recruiting participants for clinical trials of disease-modifying agents is enhanced by providing specific biomarkers.

Particular and preferred aspects of the invention are set out in the accompanying independent and dependent claims. Features from the dependent claims may be combined with features of the independent claims and with features of other dependent claims as appropriate and not merely as explicitly set out in the claims.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

Fig. 1 is a schematic diagram of a method for building and using an atlas of health-condition-associated electrophysiological phenotypes obtained from a neuronal circuit model according to embodiments of the present invention.
Fig. 2 is an overview of techniques to control the neuronal circuit architecture on chip according to embodiments of the present invention.
Fig. 3 is an overview of neuronal circuit challenges to evoke neuronal network activity according to embodiments of the present invention.
Fig. 4 is a schematic diagram of a spike sorting pipeline to derive individual neuron contributions from electrophysiological recording data according to embodiments of the present invention.
Fig. 5 is a schematic diagram of the build and use case of the atlas of health-condition-associated electrophysiological phenotypes according to embodiments of the present invention.
Fig. 6 is a flow chart of methods according to embodiments of the present invention.
Fig. 7 is a block diagram depicting a system according to embodiments of the present invention.

In the different figures, the same reference signs refer to the same or analogous elements.

### Detailed description of Illustrative Embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

The following terms are provided solely to aid in the understanding of the invention.

As used herein, the term "different types of cells" refers to cells that are distinguishable from one another based on one or more of their phenotypic characteristics. In particular, such cells may include, but are not limited to, cells of distinct lineages (e.g., neuronal and non-neuronal cells), cells exhibiting different electrophysiological properties, and cells expressing distinct sets of molecular markers. For example, within a neuronal circuit, "different types of cells" may encompass excitatory neurons, inhibitory neurons, modulatory neurons, as well as supporting cells such as glial cells.

As used herein, and unless otherwise specified, the term "neuronal circuit" refers to an arrangement of interconnected cells comprising at least two distinct cell types, at least one of which being a neuronal cell type, where these cells communicate with each other (for example, via synaptic transmission) to establish dynamic network activity (e.g., neuronal activity) within the circuit. This circuit may mimic the functional properties of neural networks found in biological organisms. Examples include cultures of neurons arranged to form synaptic connections or culture that combines neuronal cells with non-neuronal cells (such as glial cells or muscle cells) on a multi-electrode array, creating a functional network that can be stimulated and recorded.

As used herein, and unless otherwise specified, the term "multi-electrode array" refers to a device comprising an array of multiple electrodes designed to record electrical activity from cells (such as neuronal cells) placed in proximity to or in contact with the electrodes. The multi-electrode array may allow simultaneous recording from multiple electrogenic cells (e.g., neurons) to capture spatial and temporal patterns of neuronal activity. An example includes a planar complementary-metal-oxide-semiconductor (CMOS) electrode array comprising electrodes arranged in a grid pattern.

As used herein, and unless otherwise specified, the term "health condition" refers to any disease, disorder, or physiological state that affects the normal biological function or homeostasis of an organism. Particularly relevant are health conditions associated with alterations in neuronal activity or function. Examples of health conditions include neurodegenerative disorders such as Parkinson's disease, Alzheimer's disease, Huntington's disease, amyotrophic lateral sclerosis, psychiatric disorders, metabolic disorders, or any pathological state impacting neural circuits.

As used herein, and unless otherwise specified, the term "cell-level electrophysiological features" refers to characteristics of electrical activity measured from individual cells (e.g., neuronal cells) such as action potential waveform characteristics, spike amplitude, spike duration, firing rates of single electrogenic cells (e.g., neurons), or ion channel activity. Specific examples include measurements of action potential shapes, inter-spike intervals, or single-cell firing patterns.

As used herein, and unless otherwise specified, the term "circuit-level electrophysiological features" refers to characteristics of electrical activity that describe the interactions and collective behavior of a network of cells (e.g., neuronal cells). This includes parameters that capture the dynamics of the neuronal circuit as a whole, such as network synchrony, rhythmicity, connectivity patterns, and firing patterns across multiple electrogenic cells (e.g., neurons). Specific examples include measurements of network bursting activity, oscillatory behavior, synaptic connectivity maps, or overall circuit excitability.

As used herein, and unless otherwise specified, the term "biomarker" refers to a measurable characteristic or parameter that serves as an indicator of a biological state or condition, particularly one associated with the presence or progression of a health condition. In the context of the present invention, a biomarker is an electrophysiological feature derived from neuronal recordings indicative or predictive of a specific health condition. Examples include specific patterns of neuronal firing, altered synchronization in a neuronal network, or changes in action potential waveforms associated with a disease state.

As used herein, and unless otherwise specified, the phrase "composition that comprises at least two different types of cells communicating with each other and comprising at least one type of neuronal cell" means that the neuronal circuit includes a mixture of cells from at least two distinct cell types-of which at least one is a neuronal cell type-that are capable of engaging in intercellular communication (for example, through synaptic transmission or other signaling mechanisms).

As used herein, and unless otherwise specified, the phrase "composition that comprises at least two different types of neuronal cells communicating with each other " means that the neuronal circuit includes a mixture of neuronal cells of at least two distinct types capable of forming functional synaptic connections and communicating through neurotransmission. These different neuronal cell types enhance the complexity and functionality of the circuit. Examples include circuits comprising excitatory glutamatergic neurons and inhibitory GABAergic neurons, or circuits including excitatory neurons and dopaminergic modulatory neurons.

As used herein, and unless otherwise specified, the phrase "structure formed by a specific spatial arrangement of its cells on the multi-electrode array" refers to the deliberate positioning or patterning of cells on the array to create a defined architecture or organization. This organization can, for instance, mimics particular neural circuits or enhance functional connectivity. Examples include arranging neurons to replicate cortical layers, creating microcircuits resembling basal ganglia pathways, or utilizing microfabrication techniques to pattern cells in specific geometries.

As used herein, and unless otherwise specified, the term "confounding factors" refers to variables or conditions that may influence electrophysiological measurements or the association between electrophysiological features and the health condition, potentially leading to biased or misleading results. These factors are unrelated variables that can affect neuronal activity or data analysis. Examples include genetic variations, environmental exposures, differences in experimental conditions, patient age, concurrent diseases, pharmaceutical use, or lifestyle factors such as diet.

As used herein, and unless otherwise specified, the phrase "generative models and/or latent space models" refers to computational modeling approaches used to analyze complex data by learning underlying structures or distributions. They can account for confounding factors in the data. Generative models aim to model how data is generated, while latent space models involve mapping data into a lower-dimensional representation capturing essential features. Examples include variational autoencoders, principal component analysis, or other dimensionality reduction techniques used in machine learning to analyze electrophysiological data.

As used herein, and unless otherwise specified, the term "variational autoencoder schemes" refers to a type of generative model in machine learning that encodes high-dimensional data into a lower-dimensional latent space and decodes it back. This facilitates data analysis and allows handling confounding factors. These schemes can learn efficient representations of data and extract meaningful electrophysiological features while reducing noise or variability due to confounders. Examples include using VAEs to model neuronal spike data and disentangle disease-specific features from unrelated variability.

As used herein, and unless otherwise specified, the term "mutual nearest neighbor models" refers to computational methods used for data integration or batch correction by identifying nearest neighbor pairs between datasets in a shared feature space, aligning the datasets while preserving biological variability. Such models can be used to account for confounding factors by aligning electrophysiological data from different neuronal circuits or experimental batches. Examples include algorithms used to correct for batch effects or to merge datasets from different sources.

As used herein, and unless otherwise specified, the phrase "cells stem from a same first individual organism" means that the cells used to construct the neuronal circuit are derived from biological material obtained from a single individual organism, ensuring genetic consistency within the circuit. In other words, the cells originate from one donor. Examples include neuronal cells derived from induced pluripotent stem cells (iPSCs) reprogrammed from fibroblasts or blood cells of a single human donor.

As used herein, and unless otherwise specified, the phrase "use of pharmaceuticals" refers to the administration or consumption of drugs or medications by the individual organism from whom the cells are derived. This may influence the electrophysiological properties of the cells and act as confounding factors in analyses. Examples include the use of antidepressants, antipsychotics, neuroleptics, or other medications that affect neuronal function.

As used herein, and unless otherwise specified, the term "organism-centered data" refers to information specific to the individual organism from which the cells are derived, encompassing genetic, physiological, medical, and lifestyle information. These may impact the properties of the neuronal circuit. Examples include the individual's genetic information (e.g., genome sequence, presence of mutations), medical records (e.g., health history, diagnoses), concurrent diseases, use of pharmaceuticals, and diet.

As used herein, and unless otherwise specified, the term "Associated Data" refers to the dataset generated by correlating one or more electrophysiological features-derived from neuronal circuit recordings (including both cell-level and circuit-level features)-with the presence or absence of a specified health condition in the individual organism from which the neuronal circuit is derived. This dataset may also incorporate additional contextual or confounding information (such as genetic markers, clinical history, concurrent diseases, pharmaceutical usage, or dietary factors) that could influence the electrophysiological measurements. This provides an integrated record that links measured neuronal activity to the health condition under investigation.

As used herein, "Reference Associated Data" refers to a benchmark dataset constructed in a manner analogous to the Associated Data. In this case, one or more predetermined or reference electrophysiological features are correlated with the presence or absence of the same health condition. For instance, these one or more predetermined or reference electrophysiological features may be obtained from a control group or from neuronal circuits of individuals with known health statuses. This dataset serves as a standard or point of comparison for the experimental Associated Data, facilitating the identification of electrophysiological features that can reliably serve as biomarkers specific to the health condition.

The invention will now be described by a detailed description of several embodiments of the invention. It is clear that other embodiments of the invention can be configured according to the knowledge of persons skilled in the art without departing from the technical teaching of the invention, the invention being limited only by the terms of the appended claims.

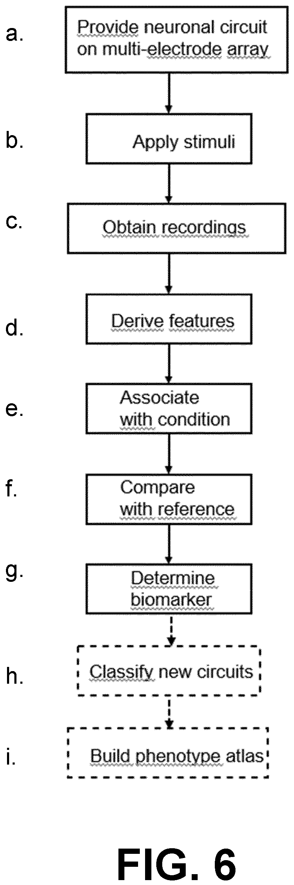

In a first aspect, as illustrated in Figure 6, the present invention relates to a method for identifying biomarkers of a health condition. The method comprises providing a first neuronal circuit for establishing neuronal activity therein, wherein all cells within the first neuronal circuit stem from the same first individual organism. The first neuronal circuit is provided on a multi-electrode array (2) for recording neuronal activity, has a composition that comprises at least two different types of cells communicating with each other and comprising at least one type of neuronal cell, and has a structure formed by a specific spatial arrangement of its cells on the multi-electrode array (2).

After step (a) of providing a first neuronal circuit, the method further comprises (b) applying one or more stimuli to the first neuronal circuit, (c) obtaining one or more electrophysiological recordings from the first neuronal circuit in response to the stimuli, (d) deriving one or more cell-level electrophysiological features and/or circuit-level electrophysiological features from at least one of the electrophysiological recordings, and (e) associating at least one derived electrophysiological feature with the presence or absence of the health condition, thereby forming associated data. The associated data are (f) compared with at least one reference associated data, formed by associating a reference electrophysiological feature with the presence or absence of the health condition. At least one of the associated data and the reference associated data is formed by associating at least one electrophysiological feature with the presence of the health condition. From that comparison, it is (g) determined whether the reference electrophysiological feature or the derived electrophysiological features are biomarkers specific for the health condition.

The combination of steps (f) and (g) corresponds to analyzing the associated data against at least one reference associated data formed by associating a reference electrophysiological feature with the presence or absence of the health condition, wherein at least one of the associated data and the reference associated data is formed by associating at least one electrophysiological feature with the presence of the health condition, to determine whether the reference electrophysiological feature or the derived electrophysiological features are biomarkers specific for the health condition. So, in an alternative formulation of the first aspect, after step (e), a step (f') may be performed of analyzing the associated data against at least one reference associated data formed by associating a reference electrophysiological feature with the presence or absence of the health condition, wherein at least one of the associated data and the reference associated data is formed by associating at least one electrophysiological feature with the presence of the health condition, to determine whether the reference electrophysiological feature or the derived electrophysiological features are biomarkers specific for the health condition.

In embodiments, the method may comprise the following steps: providing an on-chip patient-specific biological neuronal circuit model (a); applying a predetermined stimulation challenge to the MEA (b); recording electrophysiological signals from a plurality of electrodes on the MEA to capture at least the stimulated cells, wherein the recording includes at least part of the duration of the stimulation (c); extracting individual cell-level features and/or cell-cell-level features from the recorded signals (d); associating at least one derived electrophysiological feature, with the presence or absence of said health condition, thereby forming associated data (e), comparing the associated data with a reference associated data (e) wherein at least one of the associated data and the reference associated data is formed of an association of at least one electrophysiological feature with the presence of said health condition; optionally repeating steps (b) to (e) with predetermined series of stimulation challenges that can differ from each other; and combining the extracted features with a predetermined reference database so that extracted features can be compared to reference features, wherein at least one of the associated data and the reference associated data is formed of an association of at least one electrophysiological feature with the presence of said health condition (f). Next, the method comprises determining from that comparison whether at least one of the reference electrophysiological features or at least one of the derived one or more cell-level electrophysiological features and/or one or more circuit-level electrophysiological features is a biomarker specific for the health condition (g).

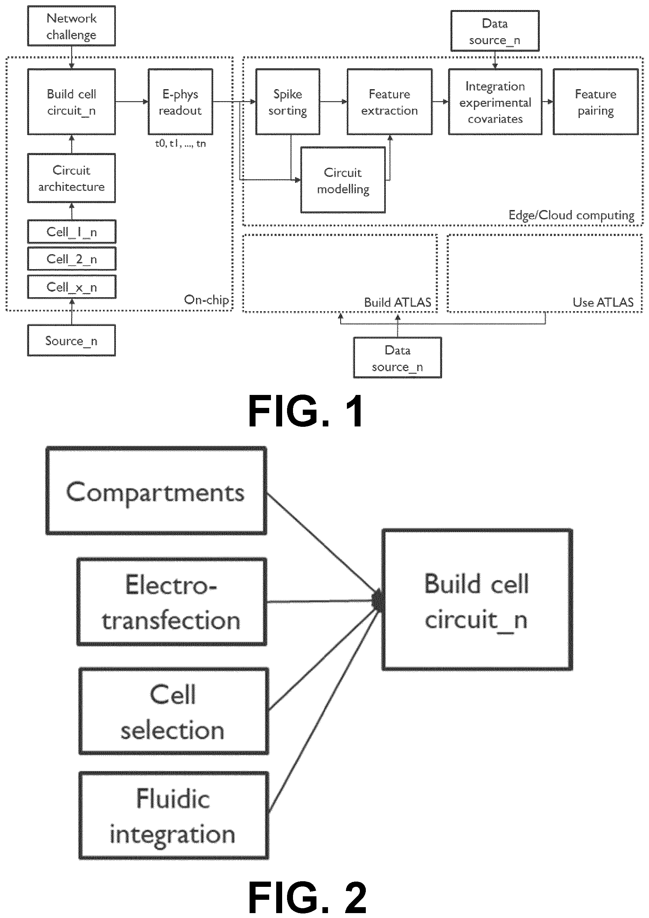

As illustrated in Figure 1, the method may involve creating an on-chip patient-specific biological neuronal circuit model from different types of patient-specific cells (Cell 1_n to Cell_x_n). The circuit architecture may be spatially and/or temporally controlled using a variety of techniques. A complementary-metal-oxide-semiconductor multi-electrode array (2), e.g., a complementary-metal-oxide-semiconductor multi-electrode array (CMOS-MEA) chip (2) may be used to obtain electrophysiological recordings from the neuronal circuit. The chip may measure spontaneous neuronal activity or activity triggered by one or a combination of different network challenges. The electrophysiological readouts may be performed at various time points (t0 to tn). A spike sorting algorithm may for instance be used to decompose the electrophysiological recording data into the contributions of individual electrogenic cells (e.g., neurons). The activation timepoints may be used in feature extraction algorithms to derive cell-level features and circuit-level features. Next, the electrophysiological features are associated with the presence or absence of a health condition and eventually with one or more covariates. Associated data is formed by this process. Next, the associated data is compared to reference associated data (Data source_n), e.g., stemming from patient-centered databases. Generative and latent space models may then be used to identify and correct for confounding effects of experimental covariates on the analysis and to pair the multimodal feature datasets. Electrophysiological features obtained from various patients are then compared, resulting in biomarker identification. This permits the development of an atlas of health-condition-associated biomarkers (or in other words, health-condition-associated electrophysiological phenotypes). New electrophysiological features can optionally be classified using the atlas.

In embodiments, the method steps (a) to (c), (a) to (d), (a) to (e), (a) to (f), or (a) to (g) may be repeated sequentially in an iterative manner for different neuronal circuits stemming from different organisms. Adjustments to one or more steps of the method in a currently analyzed neuronal circuit may be based on outputs from previously analyzed circuits. This adaptive and iterative approach enhances the robustness and reliability of biomarker identification.

For instance, repetition of the process with different predetermined stimulation challenges across multiple neuronal circuits allows building a comprehensive dataset (comprising associated data and reference associated data) for analysis.

For instance, steps (a) to (g) can be repeated across multiple neuronal circuits, each with different predetermined series of stimulation challenges.

Optional steps include culturing the neuronal circuit model on the MEA with individually addressable electrodes (z1); providing electrical signals in a predetermined pattern to the MEA for stimulating neuronal growth (z2); repeating step (z2) with a series of predetermined patterns to build a specific biological neuronal circuit model (z3); recording electrophysiological signals from each electrode (y1); determining cell type and location based on the recorded data (y2); determining a stimulation pattern according to a specified disease for applying one or more stimuli at least one cell (e.g., neuronal cell) on the MEA.

Step (a) comprises providing a first neuronal circuit. In embodiments, the structure of the neuronal circuit may mimic a neural circuit associated with the health condition, providing a biologically relevant model for studying the specific condition.

In embodiments, the health condition may be a neurodegenerative disorder selected from Parkinson's disease, Alzheimer's disease, Huntington's disease, and amyotrophic lateral sclerosis. This allows identifying biomarkers specific to these debilitating conditions.

Referring to Figure 2, various techniques may be employed to control the neuronal circuit architecture on the chip.

In embodiments, the method may further include steps to form the specific spatial arrangement of neuronal cells before establishing neuronal activity. This may involve forming a physical barrier to create compartments on the MEA, introducing different cell types into these compartments, and optionally removing the barrier. This precise control over spatial organization enhances the relevance of the neuronal circuit.

Referring back to Figure 2, techniques such as sequentially introducing different types of cells onto the MEA, electroporating cells with genetic factors, and using fluidic, acoustic, or chemical isolation techniques are employed to achieve the desired spatial arrangement.

In embodiments, undifferentiated cells may be introduced onto the MEA and electroporated with genetic factors that promote trans-differentiation into desired neuronal types. This enables in situ generation of specific neuronal subtypes within the circuit.

In a first method, a physical barrier may be used to create different compartments on the multi-electrode array. For instance, a silicone inset with adhesive may be used to create individual wells or compartments on the chip. This inset is applied to the chip surface before seeding the different cell types (Cell_1_n to Cell_x_n). After allowing cells sufficient time to adhere to the chip surface, the inset can be removed without disturbing the culture or mixing different cell types. Connections (e.g., synaptic connections) are established between cell types (e.g., neuronal cell types) during the culture period after inset removal. In a second method, electroporation of genetic factors that promote the trans-differentiation of one type of cell type (e.g., undifferentiated cells, fibroblasts, or other cell types) into the cells of interest is used. In a third method, electroporation of genetic factors conferring antibiotic resistance is employed to sequentially introduce new cell types while removing non-electroporated cells. In a fourth method, an isolation scheme using fluidics, magnetics, acoustic, or chemical isolation techniques to separate different cell types (e.g., into distinct layers).

In embodiments, undifferentiated cells may be introduced onto the MEA and electroporated with genetic factors that promote trans-differentiation into desired neuronal types. This enables in situ generation of specific neuronal subtypes within the circuit.

In embodiments, the cells may be human or mammalian. This ensures the relevance of the model to human health conditions. These cells may be derived from induced pluripotent stem cells (iPSCs), allowing for the generation of patient-specific neuronal circuits. The cells may be reprogrammed from fibroblasts or blood cells derived from the individual organism, enabling the creation of personalized neuronal circuits from accessible patient samples.

In embodiments, the at least two different types of cells communicating with each other and comprising at least one type of neuronal cell may comprise or consist of at least two types of neuronal cells selected from excitatory neurons, inhibitory neurons, and modulatory neurons, preferably comprising excitatory and modulatory neurons. This captures key neuronal subtypes involved in neural circuit function. The neuronal circuit may be supported by glial cells, providing a more physiologically relevant environment.

The composition of the circuit may comprise at least three different types of cells (e.g., neuronal cells) communicating with each other, allowing for complex and realistic neuronal architectures. These may include excitatory neurons, inhibitory neurons, and modulatory neurons, reflecting main neuronal subtypes involved in neural function.

In embodiments, excitatory neurons may comprise cortical neurons, the predominant excitatory neuron type in the cerebral cortex. Inhibitory neurons may comprise striatal neurons, predominant in the striatum. Modulatory neurons may comprise dopaminergic neurons, key in various brain functions and disorders.

The first individual organism may carry a genetic mutation associated with the health condition, allowing the study of specific genetic risk factors on neuronal circuit function.

The MEA may be a complementary-metal-oxide-semiconductor multi-electrode array chip. This proves a high-density, high-resolution platform for recording neuronal activity. It may be adapted for single-cell level recordings. This allows for precise characterization of individual neuronal responses. The MEA may have an electrode density exceeding 1000 electrodes per mm². This enables high-resolution mapping across the circuit. Electrodes may be individually addressable, allowing targeted stimulation and recording from specific regions. They may have a largest lateral dimension measuring from 2 to 500 µm, preferably from 2 to 50 µm, more preferably from 2 to 25 µm. This facilitates recording at different spatial scales.

In embodiments, the health condition may be a neurodegenerative disorder, enabling the identification of biomarkers and study of neuronal circuit dysfunction associated with conditions such as Alzheimer's disease, Parkinson's disease, or Huntington's disease.

Step (b) comprises applying one or more stimuli to the first neuronal circuit.

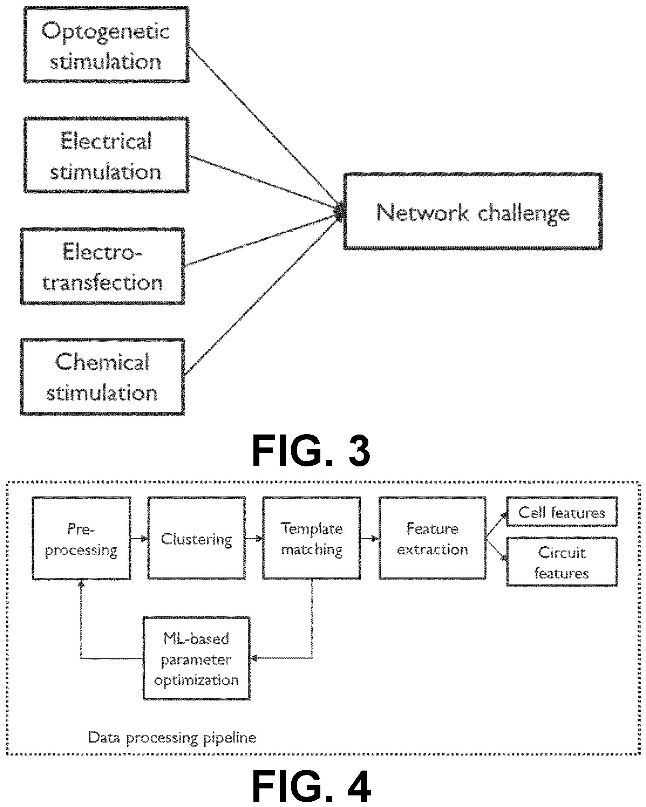

Referring to Figure 3, various neuronal circuit challenges may be employed to evoke neuronal network activity. In embodiments, network activity may be triggered by optogenetic activation of one or multiple cell phenotypes. In other embodiments, network activity may be induced by electrical stimulation of one or multiple cell phenotypes. In other embodiments, electroporation of one or multiple cell phenotypes with genetic factors may be performed to introduce or eliminate one or more mutations in various genes or combinations thereof. In embodiments, chemical factors, such as agonists or antagonists, may be introduced to evoke a neuronal response.

Hence, in embodiments, step (b) can comprise optogenetic activation, electrical stimulation, transfection, and/or chemical introduction.

In embodiments, at least one stimulus applied to the first neuronal circuit may comprise sub-steps. This allows for more complex and nuanced stimulation protocols.

As shown in Figure 3, these protocols may include combinations of optogenetic activation, electrical stimulation, genetic manipulation, and chemical factors.

In embodiments, applying one or more stimuli to the first neuronal circuit may comprise applying a plurality of stimuli to the first neuronal circuit in a predetermined sequence, obtaining one or more electrophysiological recordings from the first neuronal circuit in response to the stimuli may comprise obtaining one or more electrophysiological recordings after the application of each stimulus in said predetermined sequence, and deriving one or more cell-level electrophysiological features and/or one or more circuit-level electrophysiological features from at least one of the electrophysiological recordings may comprise deriving a cell-level electrophysiological feature and/or a circuit-level electrophysiological features from all these recordings. This enables a systematic and comprehensive evaluation of the neuronal circuit's response to a range of stimuli.

Step (c) comprises obtaining one or more electrophysiological recordings from the first neuronal circuit in response to the stimuli.

In embodiments, specific electrodes of the multi-electrode array used for obtaining one or more electrophysiological recordings may be selected using an algorithm that identifies regions of correlated neuronal activity. This optimizes data collection. The method may include performing preliminary electrophysiological recordings, before applying one or more stimuli, to detect areas on the MEA where electrogenic cells (e.g., neuronal cells) exhibit correlated firing patterns indicative of functional connectivity. An algorithm may analyze these initial data to select specific electrodes for subsequent detailed recordings, ensuring that the most relevant neuronal interactions are captured. For example, computational analysis of initial spike train data may be utilized to identify pairs or groups of electrogenic cells (e.g., neuronal cells) with statistically significant correlated firing. Electrodes associated with these electrogenic cells (e.g., neuronal cells) may be selected for high-resolution recording and analysis. This targeted selection process may be applied consistently across different neuronal circuits (e.g., including those modeling various genetic mutations) to obtain reference associated data. This allows obtaining objective and comparable datasets for biomarker discovery.

In embodiments, the composition of the neuronal circuit may be predicted based on electrophysiological recordings, and this predicted composition may be used as a quality control step to verify that the neuronal circuit matches the intended design. The method may include deriving electrophysiological features from recordings and using these features in a predictive model to estimate the types and proportions of cells (e.g., neuronal cells) present in the circuit. Deviations between the estimated composition and the intended composition may be identified and used to adjust subsequent experiments or analyses. For example, machine learning algorithms may be applied to electrophysiological features-such as action potential waveforms and spike train patterns-to predict the presence and relative abundance of excitatory neurons, inhibitory neurons, and modulatory neurons within the neuronal circuit. If the predicted cell-type composition differs significantly from the intended composition, the method may include steps to adjust the neuronal circuit construction or to recalibrate analysis parameters to ensure accurate biomarker identification.

In embodiments, steps (c) may comprise recording electrophysiological signals from a plurality of electrodes on the MEA to capture at least the electrogenic cells (e.g., neuronal cells) stimulated in step (b). This recording may include at least part of the duration of the stimulation.

Step (d) of the method comprises deriving one or more cell-level electrophysiological features and/or one or more circuit-level electrophysiological features from at least one of the electrophysiological recordings.

In embodiments, deriving one or more cell-level and/or circuit-level electrophysiological features may involve analyzing spatial variations in electrophysiological activity across the neuronal circuit as recorded from different electrodes of the MEA, analyzing temporal variations in electrophysiological activity over multiple time points, and/or analyzing spatiotemporal patterns resulting from the combination of spatial and temporal variations. This comprehensive characterization captures neuronal circuit activity at both the single-cell and network levels.

As illustrated in Figure 4, a spike sorting pipeline may be used to derive individual cell (e.g., neuronal cell) contributions from electrophysiological recording data. The algorithm identifies the timepoints in the recording dataset at which individual electrogenic cells (e.g., neuronal cells) are active. Alternatively, the spike sorting step can be replaced by a biophysical model-based fitting process to find the activation timepoints. Processing parameters may be optimized in a closed-loop optimization step that involves machine learning. Experimental covariates, such as batch identifiers and experimental workflows, may be used to facilitate the optimization process. The spike sorting pipeline and biophysical model-based fitting processes are advantageous for deriving accurate electrophysiological features.

In embodiments, deriving electrophysiological features from at least one of the recordings may comprise deriving the features from that recording and from recordings performed before applying the stimuli. This captures both the baseline activity and the evoked response of the neuronal circuit.

In embodiments, the electrophysiological features may comprise cell-level features derived from action potential waveform characteristics. This allows characterizing individual neuronal firing properties. They may include circuit-level features derived from spike train data. This allows analyzing network-level activity patterns. Features may be selected from network synchrony, rhythmicity, connectivity, and firing patterns. This allows capturing key aspects of neuronal circuit dynamics.

The electrophysiological features may be derived using a spike sorting algorithm to decompose the recording data into contributions from individual electrogenic cells (e.g., neuronal cells). This allows isolating single-unit activity. Alternatively, features may be derived using a biophysical model-based fitting process to determine activation timepoints. This allows precisely estimating neuronal firing times.

In embodiments, the method may include a feature selection step between step (d) of deriving electrophysiological features and step (f). This reduces data dimensionality and identifies the most informative features for biomarker discovery.

Step (e) comprises associating at least one derived electrophysiological feature, with the presence or absence of said health condition, thereby forming associated data.

In embodiments, associating the derived electrophysiological features with the presence or absence of the health condition may further involve associating the features with one or more confounding factors present in the individual organism from whom the neuronal circuit stems, thereby forming the associated data. The reference associated data may also be formed by associating the reference electrophysiological feature with the same confounding factors. This approach accounts for potential confounding variables that may influence the electrophysiological features.

In embodiments, confounding factors may comprise batch-specific variability and/or experimental covariates. These account for potential sources of experimental noise or bias. The performance of comparing associated data and determining biomarkers may involve using mutual nearest neighbor models. This provides a robust approach for identifying biomarkers from high-dimensional data.

Step f comprises comparing the associated data with at least one reference associated data.

Referring back to Figure 1, generative and/or latent space models may be utilized to identify and correct for confounding effects of experimental covariates on the analysis and to pair the multimodal feature datasets (by comparing the formed associated data with at least one reference associated data). These confounding factors may include organism-centered data such as genetic information, medical records, concurrent diseases, use of pharmaceuticals, and diet.

In other words, the performance of comparing the associated data with the reference associated data and determining whether the electrophysiological features are biomarkers specific for the health condition may involve using generative models and/or latent space models to account for confounding factors. This robust approach handles confounding variables in the biomarker identification process. The generative models may include variational autoencoder schemes, offering a powerful framework for modeling complex data distributions and accounting for confounding factors.

In embodiments, one or more of the reference associated data may be obtained in the same way, e.g., by performing steps (a) to (e) in exactly the same way, as the associated data but by providing a neuronal circuit stemming from an individual different from the first individual. This controlled comparison between neuronal circuits from different individuals maintains consistency in experimental conditions.

Reference associated data can come from various sources such as databases and experiments. In the context of the present invention, any embodiment related to the formation of associated data, i.e., any embodiment of steps (a) to (e), may be applied for the formation of reference associated data. In fact, as soon as method steps (a) to (e) have been performed at least twice on circuits stemming from at least two different individual organisms, one has de facto obtained associated data and reference associated data.

This means that, in embodiments, the method for identifying biomarkers of a health condition may comprise:
(a) providing a first neuronal circuit for establishing neuronal activity therein, wherein:
   - all cells within the first neuronal circuit stem from a same first individual organism,
      wherein the first neuronal circuit:
      - is provided on a multi-electrode array (2) for recording a neuronal activity;

   - has a composition that comprises at least two different types of communicating cells communicating with each other and comprising at least one type of neuronal cell; and
   - has a structure formed by a specific spatial arrangement of its cells on the multi-electrode array (2);
(b) applying one or more stimuli to the first neuronal circuit,
(c) obtaining one or more electrophysiological recordings from the first neuronal circuit in response to the stimuli;
(d) deriving one or more cell-level electrophysiological features and/or one or more circuit-level electrophysiological features from at least one of the electrophysiological recordings;
(e) associating at least one derived electrophysiological feature, with the presence or absence of said health condition, thereby forming associated data,
(e') repeating steps (a) to (e) at least one time, thereby obtaining a series of associated data, wherein the series of associated data comprises associated data formed of the association of a electrophysiological feature with the presence of said health condition and associated data formed of the association of a electrophysiological feature with the absence of said health condition,
(f') determine from an analysis of the series of associated data whether one or more of the electrophysiological features of the associated data is a biomarker specific for the health condition.

Machine learning classification algorithms, such as decision trees, logistic regression, principal component analysis, naive Bayes models, support vector machines, random forests, and nearest neighbor models, may be applied in the analysis, i.e., in steps (f) and (g). This utilizes powerful computational tools for biomarker identification.

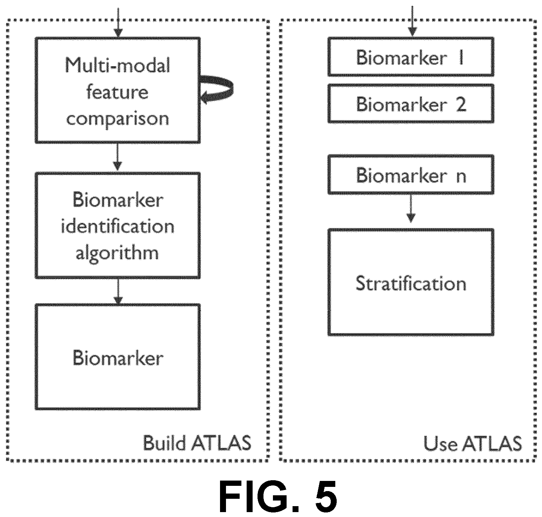

Referring to Figure 5, electrophysiological features obtained from various patients may be compared and classified between groups. A feature selection step may be applied to reduce data dimensionality and facilitate classification. Various classification schemes may be used to process electrophysiological features, e.g., for the introduction or elimination of disease subgroups, for estimating the probability of disease subtypes, and/or for biomarker identification. This allows the formation of an atlas of health-condition-associated electrophysiological phenotypes.

In embodiments, the method steps may be repeated sequentially in an iterative manner for different neuronal circuits. Adjustments to one or more steps of the method in a currently analyzed neuronal circuit may be based on outputs from previously analyzed circuits. This adaptive and iterative approach enhances the robustness and reliability of biomarker identification.

The method may be used for identifying biomarkers of a health condition and classifying neuronal circuits. It may further involve obtaining electrophysiological recordings from new individual organisms' neuronal circuits, extracting features, comparing them to identified biomarkers, and classifying the circuits into phenotypically different populations. This enables the application of identified biomarkers for diagnostic or prognostic purposes.

In embodiments, the method may be used for phenotype atlas construction. Building an atlas of disease-associated electrophysiological phenotypes based on identified biomarkers creates a comprehensive resource for understanding electrophysiological signatures of different health conditions.

Referring back to Figures 1 and 5, the development of an atlas involves comparing and classifying electrophysiological features from various patients. This atlas serves as a valuable tool for biomarker identification and understanding the diversity of disease phenotypes.

In a second aspect, the present invention relates to a system for identifying biomarkers of a health condition, comprising means adapted to execute the steps of the method described in any embodiment of the first aspect.

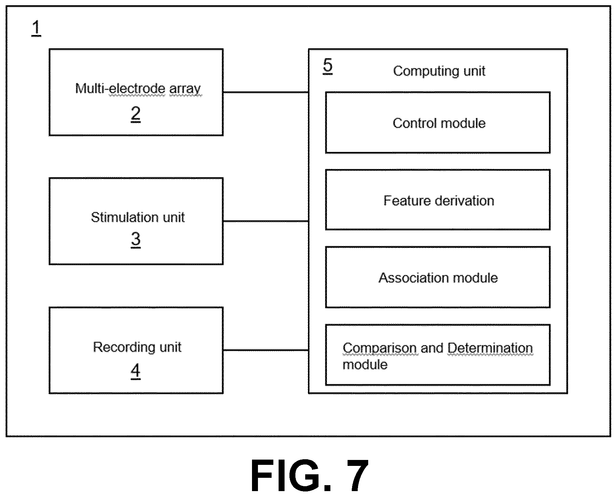

Referring to Figure 7, the system (1) according to the second aspect of the present invention may comprise a multi-electrode array (MEA) (2) configured to support a neuronal circuit, a stimulation unit (3) configured to apply one or more stimuli to evoke neuronal activity, a recording unit (4) configured to obtain electrophysiological recordings in response to the stimuli, and a computing unit (5) configured to control the stimulation and recording units, derive cell-level and/or circuit-level electrophysiological features, associate each derived feature with the presence or absence of a health condition and with confounding factors to form associated data, compare the associated data with reference associated data, and determine from that comparison whether the electrophysiological features are biomarkers specific for the health condition.

In embodiments, the stimulation unit may apply stimuli selected from acoustic stimuli, optical stimuli such as optogenetic stimuli, electrical stimuli, chemical stimuli including biochemical stimuli, electroporation of genetic factors into cells (e.g., neuronal cells), or combinations thereof. This enables evoking different types of neuronal activity.

In embodiments, the multi-electrode array (2) may be a complementary-metal-oxide-semiconductor multi-electrode array chip, providing high spatial resolution. The multi-electrode array (2) may be adapted for single-cell level recordings of neuronal activity, allowing derivation of cell-level features. The multi-electrode array (2) may have an electrode density exceeding 1,000 electrodes per mm², enabling high-resolution mapping. The electrodes may be individually addressable, allowing targeted stimulation and recording. The electrodes' largest lateral dimensions may range from 2 to 500 µm, preferably from 2 to 50 µm, more preferably from 2 to 25 µm, which is optimal for interfacing with neurons.

In embodiments, the electrophysiological features may include cell-level features derived from action potential waveform characteristics, providing information on cell firing properties. Circuit-level features may be derived from spike train data, offering insights into network dynamics. These circuit-level features may be selected from network synchrony, rhythmicity, connectivity, and firing patterns, which are key aspects of circuit function.

The computing unit may be configured to use a spike sorting algorithm to decompose the electrophysiological recording data into contributions from individual electrogenic cells (e.g., neuronal cells), enabling cell-level analysis. Alternatively, it may use a biophysical model-based fitting process to determine activation timepoints. The computing unit may form and compare the associated data using generative models and/or latent space models to account for confounding factors, enabling robust comparisons. Generative models may include variational autoencoder schemes and mutual nearest neighbor models.

The computing unit may form and compare the associated data by applying machine learning classification algorithms selected from decision trees, logistic regression, principal component analysis, naive Bayes models, support vector machines, random forests, and nearest neighbor models. These are established classification techniques.

In embodiments, the computing unit may repeat the identification of biomarkers for different neuronal circuits and adjust one or more steps of the method in a currently analyzed neuronal circuit based on the output of one or more steps from previously analyzed neuronal circuits. This enables iterative optimization.

A feature selection step may be applied by the computing unit between the derivation of features and forming the associated data to reduce data dimensionality during analysis, facilitating classification.

Once biomarkers have been identified, the computing unit may compare extracted features derived from an individual organism's neuronal circuit to the identified biomarkers. Based on that comparison, it may classify the individual organism's neuronal circuit into a phenotypically specific population, enabling patient stratification.

The computing unit may further build an atlas of disease-associated electrophysiological phenotypes based on the identified biomarkers, providing a reference for diagnosis.

In a fourth aspect, the present invention relates to a computer program comprising instructions to cause the system described in any embodiment of the third aspect to execute the steps of the method described in any embodiment of the first aspect.

In a fifth aspect, the present invention relates to a computer-readable medium having stored thereon the computer program described in the fourth aspect.

### Example 1: Method for Identifying Dopamine Signaling-Related Biomarkers in a Human iPSC-Derived Cortico-Striato-Nigral Circuit on Multi-Electrode Arrays

A first neuronal circuit is provided by differentiating these iPSCs into three distinct neuronal cell types: cortical excitatory neurons, striatal medium spiny neurons, and nigral dopaminergic neurons. These differentiated neuronal cells, all originating from the same individual organism, are arranged in a specific spatial pattern on a high-density multi-electrode array containing 26,400 electrodes spaced 17.5 µm apart. The spatial arrangement consists of three distinct regions on the array, with each region containing a different neuronal cell type, allowing for the formation of communicating networks between these neuronal populations. The circuit architecture is spatially controlled by applying a silicone inset with adhesive on the chip surface to create individual compartments before seeding the different cell types. After allowing sufficient time for the cells to adhere, the inset is removed without disturbing the culture or mixing the cell types. Connections (e.g., synaptic connections) are established between the cell types (e.g., neuronal cell types) during the subsequent culture period.

The stimulus applied to the first neuronal circuit consists of the selective blockade of D1 dopamine receptors using the antagonist SCH23390 at a concentration of 0.5 µM.

Electrophysiological recordings are obtained both before and after the application of this stimulus, capturing the electrical activity across all three neuronal populations simultaneously at a sampling rate of 10 kHz.

From these recordings, multiple electrophysiological features are derived at both the cell and circuit levels. A spike sorting algorithm is used to decompose the electrophysiological recording data into the contributions of individual cells (e.g., electrogenic cells such as neuronal cells) by identifying the timepoints at which each cell (e.g., neuron) is active. The identified activation timepoints are used to derive cell-level features from representative waveforms and circuit-level features by fitting statistical or mechanistic models to the spike train data. The cell-level features include firing rates, spike waveform characteristics, and burst properties of individual electrogenic cells (e.g., neuronal cells). The circuit-level features encompass network synchronization, burst frequency, and population-wide firing patterns.

These derived electrophysiological features are then associated with Parkinson's disease, a known condition of the cell donor, thereby forming the associated data.

This associated data is compared with reference associated data obtained from the same protocol operated on cells from a healthy donor.

The comparison reveals that specific electrophysiological features, particularly the changes in neuronal excitability patterns and an increase in circuit synchronicity, can serve as potential biomarkers for the compromised dopamine signaling characteristic of Parkinson's disease.

### Example 2: Alzheimer's Disease Biomarker Identification

A plurality of neuronal circuits is created on a corresponding number of CMOS-MEA chips using induced pluripotent stem cell (iPSC)-derived neurons from patients with early-stage Alzheimer's disease and age-matched healthy controls. The circuit architecture is controlled using a silicone inset with adhesive to create separate compartments for different neuron types, including, in this example, hippocampal neurons originating from the dentate gyrus, hippocampal neurons originating from the CA1 field, and astrocytes. After removal of the inset, synaptic connections are established between the neuron types.

Electrophysiological recordings are obtained under various conditions, including spontaneous activity and activity triggered by electrical stimulation of one part of the circuit. A spike sorting algorithm is used to identify individual neuron contributions, and both cell-level and circuit-level features are extracted from the data.

The extracted features are combined with patient genetic data, specifically focusing on APOE genotype and other known Alzheimer's risk genes. Generative models are used to account for confounding factors such as age and sex. Classification algorithms, including support vector machines and random forests, are applied to identify electrophysiological biomarkers specific to early-stage Alzheimer's disease.

### Example 3: Parkinson's Disease Subtype Classification

A brain-on-a-chip model is developed using iPSC-derived dopaminergic neurons, medium spiny neurons, and astrocytes from patients with different subtypes of Parkinson's disease (tremor-dominant, postural instability and gait difficulty, and mixed) and healthy controls. The neuronal circuit architecture is controlled using electroporation of genetic factors to promote trans-differentiation of fibroblasts into the desired neuron types.

Electrophysiological recordings are obtained using optogenetic activation of the dopaminergic neurons and chemical stimulation with dopamine receptor agonists and antagonists. Cell-level features are derived from action potential waveform characteristics, while circuit-level features focus on connectivity and rhythmicity.

The electrophysiological features are integrated with patient data on motor symptom severity, non-motor symptoms, and response to levodopa treatment. Variational autoencoders are used to account for confounding factors such as disease duration and medication history.

Machine learning algorithms, including decision trees and naive Bayes models, are applied to analyze the series of associated data, to determine biomarkers specific for subtypes of Parkinson's disease.

### Example 4: Amyotrophic Lateral Sclerosis (ALS) Progression Monitoring

A longitudinal study is conducted using neuronal circuits created from iPSC-derived motor neurons and myocytes from ALS patients with different rates of disease progression. The circuit architecture is controlled using a mechanical isolation scheme to separate motor neurons and astrocytes into different layers. Electrophysiological recordings are obtained at multiple time points over several months, with network activity induced by electrical stimulation and introduction of glutamate to mimic excitotoxicity. Both spontaneous and evoked activity are recorded and analyzed.

In the present example, cell-level features focus on motor neuron action potential characteristics and firing rates, while circuit-level features examine network synchrony and connectivity. These electrophysiological features are combined with microscopic information on axon length, stability, and retraction, with patient data on functional decline (ALSFRS-R scores) and with biomarkers of neurodegeneration (neurofilament light chain levels).

Principal component analysis and support vector machines are used to identify electrophysiological signatures associated with rapid disease progression.

### Example 5: Identification of Alpha-Synuclein Fibril-Induced Electrophysiological Biomarkers in a Human Cortico-Striato-Nigral Circuit

A first neuronal circuit was provided on a complementary-metal-oxide-semiconductor multi-electrode array (MEA). All cells within this circuit were derived from a single human donor. The circuit was composed of human induced pluripotent stem cell (hiPSC)-derived cortical excitatory neurons, striatal medium spiny neurons, and nigral dopaminergic neurons that were spatially arranged in a defined pattern on the MEA to mimic the anatomical organization of the cortico-striato-nigral pathway.

Subsequently, one or more stimuli were applied to the circuit. In this instance, the circuit was inoculated with sonicated, fluorescently-tagged alpha-synuclein fibrils at a concentration of 1 µg/mL. This treatment served as a Parkinson's disease-relevant pathological challenge by introducing misfolded alpha-synuclein into the circuit.

Electrophysiological recordings were obtained from the circuit both before and after the inoculation. Recordings were performed at baseline and at several time points post-inoculation (specifically at 3, 7, 14, and 28 days) using the high-density MEA. These recordings captured the spontaneous and network-level neuronal activity of the circuit.

From the electrophysiological recordings, a set of cell-level and circuit-level features was derived. These features included single-cell firing rates, spike waveform characteristics, burst activity parameters, and network synchronization metrics. A spike sorting algorithm was applied to decompose the recorded data into individual neuron activities, thereby allowing accurate extraction of these parameters.

The derived electrophysiological features were then associated with the presence of the alpha-synuclein fibril-induced condition. In this process, the changes in network synchronization and burst dynamics observed after fibril inoculation were correlated with the treatment condition. This association formed the "associated data," linking the electrophysiological signatures with the induced pathological state.

The associated data from the fibril-treated circuits were compared with reference associated data obtained from control circuits that had not been inoculated with alpha-synuclein fibrils. Comparative analyses, which included the use of machine learning classifiers (such as a random forest model), were performed to identify differences in the electrophysiological features between the treated and control groups.

From the comparison, it was determined that certain electrophysiological features-most notably, an increase in network synchronization and altered burst patterns-served as biomarkers specific for the alpha-synuclein fibril-induced condition. These biomarkers were validated by the classifier's high prediction accuracy in distinguishing between fibril-treated and untreated circuits, thereby confirming that the observed network-level changes were indicative of the pathological effects associated with misfolded alpha-synuclein.

Additional illustrative embodiments of the invention are provided in the Examples section (table 1, Examples 1-9, materials and methods, associated Figures 1-6) of the application EP25155769 'HUMAN NEURONAL CIRCUITS'. The entirety of this Example section is hereby incorporated by reference.

It is to be understood that although preferred embodiments, specific constructions and configurations, as well as materials, have been discussed herein for devices according to the present invention, various changes or modifications in form and detail may be made without departing from the scope of this invention. For example, any formulas given above are merely representative of procedures that may be used. Functionality may be added or deleted from the block diagrams and operations may be interchanged among functional blocks. Steps may be added or deleted to methods described within the scope of the present invention.

### List of Reference Numbers

1. system
2. multi-electrode array
3. stimulation unit
4. recording unit
5. computing unit

## Claims

1. A method for identifying biomarkers of a health condition, the method comprising:
(a) providing a first neuronal circuit for establishing neuronal activity therein, wherein:
- all cells within the first neuronal circuit stem from a same first individual organism,
wherein the first neuronal circuit:
- is provided on a multi-electrode array (2) for recording a neuronal activity;
- has a composition that comprises at least two different types of cells communicating with each other and comprising at least one type of neuronal cell; and
- has a structure formed by a specific spatial arrangement of its cells on the multi-electrode array (2);
(b) applying one or more stimuli to the first neuronal circuit,
(c) obtaining one or more electrophysiological recordings from the first neuronal circuit in response to the stimuli;
(d) deriving one or more cell-level electrophysiological features and/or one or more circuit-level electrophysiological features from at least one of the electrophysiological recordings;
(e) associating at least one derived electrophysiological feature, with the presence or absence of said health condition, thereby forming associated data,
(f) comparing the associated data with at least one reference associated data formed of the association of a reference electrophysiological feature with the presence or absence of said health condition, wherein at least one of the associated data and the reference associated data is formed of an association of at least one electrophysiological feature with the presence of said health condition,
(g) determine from that comparison whether at least one of the reference electrophysiological features or at least one of the derived one or more cell-level electrophysiological features and/or one or more circuit-level electrophysiological features is a biomarker specific for the health condition.

2. The method according to claim 1, wherein step (e) further comprises associating the at least one derived electrophysiological feature with one or more confounding factors present in the individual organism from whom the neuronal circuit stems from, thereby forming the associated data, and wherein the reference associated data is further formed from the association of the reference electrophysiological feature with said one or more confounding factors.

3. The method according to any one of claims 1 or 2, wherein one or more of the at least one reference associated data are obtained in the same way as the associated data but by providing in step (a) at least one neuronal circuit stemming from an individual different from the first individual.

4. The method according to any one of the preceding claims, wherein one or more preliminary electrophysiological recordings of the first neuronal circuit are performed before applying the one or more stimulus, wherein said preliminary electrophysiological recordings are used to detect areas on the multi-electrode array (2) where neuronal cells exhibit preliminary correlated firing patterns indicative of functional connectivity, and wherein step (c) comprises obtaining one or more electrophysiological recordings from the first neuronal circuit at these areas.

5. The method according to any one of the preceding claims, wherein the structure of the neuronal circuit mimics a neural circuit associated with the health condition.

6. The method according to any one of the preceding claims, wherein the neuronal cells are human or mammalian neuronal cells.

7. The method according to any one of the preceding claims, wherein the at least two different types of communicating cells are selected from excitatory neurons, inhibitory neurons, and modulatory neurons, and preferably comprise excitatory neurons and modulatory neurons.

8. The method according to any one of the preceding claims, wherein the multi-electrode array (2) is adapted for single-cell level recordings of neuronal activity.

9. The method according to any one of the preceding claims, wherein the multi-electrode array (2) electrodes are individually addressable.

10. The method according to any one of the preceding claims, wherein steps (a) through (g) are repeated sequentially in an iterative manner for different neuronal circuits, the method further comprising adjusting one or more of steps of the method in a currently analyzed neuronal circuit, based on the output of one or more steps of the method in one or more previously analyzed neuronal circuits.

11. The method according to any one of the preceding claims, wherein the method is for identifying biomarkers of a health condition and h) classifying neuronal circuits, the method further comprising:
h1) Obtaining electrophysiological recordings from new individual organisms neuronal circuits;
h2) Extracting electrophysiological features from these recordings;
h3) Comparing the extracted features to at least one biomarker identified in step (g); and
h4) based on the comparison made in h3), classifying the new individual organism neuronal circuits into phenotypically different populations.

12. The method according to any one of the preceding claims, wherein the method is for identifying biomarkers of a health condition and for phenotype atlas construction, the method further comprising i) building an atlas of disease-associated electrophysiological phenotypes based on the identified biomarkers.

13. A system (1) for identifying biomarkers of a health condition, comprising:
(a) a multi-electrode array (2) configured to support a neuronal circuit;
(b) a stimulation unit (3) configured to apply one or more stimuli to the neuronal circuit to evoke neuronal activity therein;
(c) a recording unit (4) configured to obtain electrophysiological recordings from the neuronal circuit in response to the stimuli;
(d) a computing unit (5) configured to:
control the stimulation unit (3) and the recording unit (4),
derive one or more cell-level and/or circuit-level electrophysiological features from the electrophysiological recordings;
associate each derived electrophysiological feature with the presence or absence of a health condition in the individual organism from which the neuronal circuit stem from and with one or more confounding factors, thereby forming associated data; and
compare the associated data with at least one reference associated data formed of the association of a reference electrophysiological feature with the presence or absence of said health condition, wherein at least one of the associated data and the reference associated data is formed of an association of at least one electrophysiological feature with the presence of said health condition,
determine from that comparison whether the reference electrophysiological feature, said at least one of the derived one or more cell-level electrophysiological features and/or one or more circuit-level electrophysiological features is a biomarker specific for the health condition.

14. A computer program comprising instructions to cause the system (1) of claim 13 to execute the steps of the method of any one of claims 1 to 12.

15. A computer-readable medium having stored thereon the computer program of claim 14.
